# EUROPEAN PATENT APPLICATION

(11) **EP 3 153 139 A1**
(43) Date of publication of application: **12.04.2017**
(21) Application number: 15803562.6
(22) Date of filing: 03.06.2015
(51) Int. Cl.: A61F 9/00, A61F 2/16, F21S 2/00, G02C 7/00, G02C 7/04, F21W 131/20

(54) **MYOPIA PREVENTION ARTICLE**

(30) Priority: 03.06.2014 JP 2014115286
(71) Applicant: Tsubota Laboratory, Inc., Tokyo 107-0062 (JP)
(72) Inventor: TSUBOTA, Kazuo, Minato-ku, Tokyo 107-0062 (JP); NEGISHI, Kazuno, Minato-ku, Tokyo 107-0062 (JP); TORII, Hidemasa, Minato-ku, Tokyo 107-0062 (JP); KURIHARA, Toshihide, Minato-ku, Tokyo 107-0062 (JP)
(74) Representative: Addiss, John William
(86) International application number: PCT/JP2015/065997
(87) International publication number: WO 2015/186723

(57) **Abstract**

The purpose of the present invention is to provide a myopia prevention article. The provided myopia prevention article comprises a light transmission part through which ultraviolet light with a wavelength of more than 315 nm and not more than 400 nm or 360 nm to 400 nm inclusive is transmitted and ultraviolet light with a wavelength of 315 nm or less is not transmitted, or a light emission part which emits ultraviolet light with a wavelength of more than 315 nm and not more than 400 nm or 360 nm to 400 nm inclusive.

## Description

### Field of the Invention

The present invention relates to a myopia prevention article.

### BACKGROUND ART

The eye reportedly incurs various damage when exposed to ultraviolet light (Saito, et al., Jpn Opthalmol 2010; 54 486-493, Per G. Soderberg, Progress in Biophysics and Molecular Biology 107 (2011) 389-392). As a result, many products, such as eyeglasses and contact lenses, that minimize the transmission of ultraviolet light to the extent possible to prevent eye exposure to ultraviolet light across the full spectrum of wavelengths are now commercially available.

Meanwhile, the number of persons with myopia is reportedly still increasing worldwide. Myopia includes refractive myopia and axial myopia, many cases being axial myopia. With axial myopia, myopia progresses in association with elongation of an axial length of the eye, and the elongation is irreversible (Morgan IG, et al., Lancet, 2012). When myopia progresses, high myopia occurs, and high myopia is known as the leading cause of blindness (Iwase A, et al., Ophthalmology, 2006). For this reason, there has been strong demand for means for preventing the occurrence of myopia and means for delaying the progression of myopia.

### SUMMARY OF THE INVENTION

### Problems to be Solved by the Invention

It is therefore an object of the present invention to provide a myopia prevention article.

### Means for Solving the Problems

The present inventors were the first to discover that a degree of elongation of the axial length of an eye exposed to ultraviolet light of more than 315 nm and not more than 400 nm is significantly small compared to the degree of elongation of the axial length of an eye not exposed to ultraviolet light across the full spectrum of wavelengths, and the degree of myopia related to the refractive value of the eye decreases. That is, the present inventors were the first to discover that exposure of the eye to ultraviolet light only made of the wavelengths in that specified range makes it possible to prevent myopia and delay the progression of myopia. The present invention addresses the problem of providing a myopia prevention article that ensures eye exposure to ultraviolet light with wavelengths of more than 315 nm and not more than 400 nm or 360 nm to 400 nm inclusive.

An embodiment of the present invention is a myopia prevention article (excluding Artiflex (brand name)) that comprises a light transmission part through which ultraviolet light with a wavelength of more than 315 nm and not more than 400 nm is transmitted and ultraviolet light with a wavelength of 315 nm or less is not transmitted. The myopia prevention article may be an article that comprises a light transmission part through which ultraviolet light with a wavelength of 360 nm to 400 nm inclusive is transmitted and ultraviolet light with a wavelength of 360 nm or less is not transmitted.

Another embodiment of the present invention is a myopia prevention article that comprises a light emission part which emits ultraviolet light with a wavelength of more than 315 nm and not more than 400 nm or 360 nm to 400 nm inclusive.

Yet another embodiment of the present invention is a method for investigating if ultraviolet light with a predetermined wavelength within a range of more than 315 nm and not more than 400 nm or 360 nm to 400 nm inclusive can prevent myopia, and includes a step of irradiating ultraviolet light with a wavelength of more than 315 nm and not more than 400 nm or 360 nm to 400 nm inclusive on an animal.

Yet another embodiment of the present invention is a myopia prevention method that includes a step of irradiating ultraviolet light with a wavelength of more than 315 nm and not more than 400 nm or 360 nm to 400 nm inclusive on an animal.

Yet another embodiment of the present invention is a set of a myopia prevention article (excluding Artiflex (brand name)) that comprises the light transmission part through which ultraviolet light with a wavelength of more than 315 nm and not more than 400 nm is transmitted and ultraviolet light with a wavelength of 315 nm or less is not transmitted, or the light transmission part through which ultraviolet light with a wavelength of 360 nm to 400 nm inclusive is transmitted and ultraviolet light with a wavelength of 360 nm or less is not transmitted, and a myopia prevention article that comprises the light emission part which emits ultraviolet light with a wavelength of more than 315 nm and not more than 400 nm or 360 nm to 400 nm inclusive. The set may comprise a myopia prevention article selected from the group including an eyesight correcting tool, an eye protection tool, a face protection tool, a sunshade, a display device, a window, a wall, a light source covering, and a coating material, and a myopia prevention article selected from the group including lighting equipment, a display device, and an ultraviolet light irradiation device.

### Cross-reference with related literature

The present application claims priority on the basis of Japanese Patent Application 2014-115286 filed on June 3, 2014, the basic application of which is hereby incorporated by reference.

### BRIEF DESCRIPTION OF THE DRAWINGS

Fig. 1 is a graph showing a spectral transmission curve of an intraocular lens used in one example of the present invention.
Fig. 2 is a graph showing a spectral transmission curve of an intraocular lens used in one example of the present invention.
Fig. 3 is a graph showing degrees of elongation of an axial length of control eyes and eyes into which one of two types of intraocular lenses was inserted in one example of the present invention.
Fig. 4 is a graph showing the relationship between a strength and a wavelength of ultraviolet light emitted by a UVA irradiation device used in one example of the present invention.
Fig. 5 is a table showing the relationship between the strength of ultraviolet light emitted by the UVA irradiation device and a distance from the UVA irradiation device, used in one example of the present invention.
Fig. 6 is a graph showing refractive values of eyes of chicks irradiated with UVA and eyes of chicks not irradiated with UVA in one example of the present invention.
Fig. 7 is a graph showing axial lengths of eyes of chicks irradiated with UVA and eyes of chicks not irradiated with UVA in one example of the present invention.
Fig. 8 is a graph showing the relationship between a strength and a wavelength of short wavelength light having a peak at 305 nm and emitted by the UVA irradiation device, used in one example of the present invention.
Fig. 9 is an example of an optical spectrum of an LED lamp that does not emit short wavelength light with a wavelength of more than 315 nm and not more than 400 nm and does not have a myopia suppression effect.
Fig. 10 is an example of a transmittance light spectrum of eyeglass lenses through which short wavelength light with a wavelength of more than 315 nm and not more than 400 nm is not transmitted, and by which there is no myopia suppression effect.
Fig. 11 is an optical spectrum of a lamp that emits short wavelength light with a wavelength of more than 360 nm and not more than 400 nm in one example of the present invention.
Fig. 12 is a lamp having the optical spectrum shown in Fig. 13 in one example of the present invention.
Fig. 13 is a transmittance spectrum of eyeglass lenses through which short wavelength light with a wavelength of more than 315 nm and not more than 400 nm is transmitted in one example of the present invention.

### Embodiments of the Invention

The following describes in detail embodiments of the present invention that were completed on the basis of the discovery described above while providing examples. Note that the object, features, advantages, and ideas of the present invention are apparent to those skilled in the art from the descriptions herein. Furthermore, those skilled in the art can easily reproduce the present invention from these descriptions. The embodiments and the specific examples described below represent preferable modes of the present invention, which are given for the purpose of illustration or description. The present invention is not limited thereto. It is obvious to those skilled in the art that various changes and modifications may be made according to the descriptions of the present specification without departing from the spirit and scope of the present invention disclosed herein.

In the present specification, "to prevent myopia" means to prevent the occurrence of myopia and to delay the progression of myopia.

Ultraviolet light can be classified into UVA, UVB, and UVC according to wavelength, and the wavelength ranges thereof are from 315 nm to 400 nm for UVA, from 280 nm to 315 nm for UVB, and from 100 nm to 280 nm for UVC. The present invention prevents myopia by ensuring that the eye is exposed to ultraviolet light with a wavelength of more than 315 nm and not more than 400 nm, and preferably 360 nm to 400 nm inclusive.

In the present invention, "ultraviolet light with a wavelength of more than 315 nm and not more than 400 nm" and "360 nm to 400 nm inclusive" may be ultraviolet light across the full spectrum of wavelengths within the respective ranges, or ultraviolet light across a portion of wavelengths within the respective ranges.

### (1) Myopia prevention article comprising light transmission part

The myopia prevention article that comprises a light transmission part according to the present invention may be an article that uses a material through which ultraviolet light with a wavelength of more than 315 nm and not more than 400 nm, and preferably 360 nm to 400 nm inclusive, among the wavelengths of light such as natural light and artificial light is transmitted, and ultraviolet light with a wavelength of 315 nm or less is not transmitted for the light transmission part.

The light transmission part according to the present invention may be one part through which ultraviolet light with a wavelength of more than 315 nm and not more than 400 nm, and preferably 360 nm to 400 nm inclusive, is transmitted, and ultraviolet light with a wavelength of 315 nm or less is not transmitted, or may be a combination of a part through which ultraviolet light with a wavelength of more than 315 nm and not more than 400 nm, and preferably 360 nm to 400 nm inclusive, is transmitted and a part through which ultraviolet light with a wavelength of 315 nm or less is not transmitted.

Specific examples include eyesight correcting tools (eyeglass lenses, contact lenses, intraocular lenses, and the like), eye protection tools (sunglasses, protective glasses, goggles, and the like), face protection tools (helmet shields and the like), sunshades (umbrellas, sunvisors, and the like), display screens of display devices (televisions, PC monitors, gaming devices, portable media players, cell phones, tablet terminals, wearable devices, 3D glasses, virtual glasses, mobile book readers, car navigation systems, imaging devices such as digital cameras, monitors inside vehicles, monitors inside airplanes, and the like), curtains (cloth curtains, vinyl curtains, and the like), windows (windows of a building, vehicle, or airplane; front or rear windows; and the like), walls (glass curtain walls and the like), light source coverings (light covers and the like), and coating materials (seals, coating liquids, and the like), but the present invention is not limited thereto.

Here, the intraocular lens refers to an artificial crystalline lens inserted when a crystalline lens is removed, or a phakic intraocular lens inserted for the purpose of myopia correction.

The material of the light transmission part is not particularly limited as long as the material is capable of performing processing so that ultraviolet light with a wavelength of more than 315 nm and not more than 400 nm, and preferably 360 nm to 400 nm inclusive is transmitted, and ultraviolet light with a wavelength of 315 nm or less or 360 nm or less, respectively, is not transmitted. Examples include glass and plastic. Further, a known ultraviolet absorber, ultraviolet scattering agent, or the like may be used.

In the case of "ultraviolet light is not transmitted," the ultraviolet transmission rate is preferably 1% or less, and more preferably 0.1% or less. Methods for measuring the ultraviolet transmission rate are well known to those skilled in the art, and measurement can be performed using any known measuring device and method.

The transmission rate of ultraviolet light with a wavelength of more than 315 nm and not more than 400 nm, and preferably 360 nm to 400 nm inclusive, may be selected as appropriate according to the amount of surrounding ultraviolet light, but is preferably 21% or greater, and more preferably 30% or greater.

A strength of transmitted ultraviolet light with a wavelength of more than 315 nm and not more than 400 nm, and preferably 360 nm to 400 nm inclusive, is preferably not more than 5.0 mW/cm², more preferably not more than 3.0 mW/cm², more preferably not more than 1.0 mW/cm², more preferably not more than 0.5 mW/cm², and more preferably not more than 0.25 mW/cm². The strength can be measured using a known method.

The myopia prevention article that comprises the light transmission part according to the present invention may transmit light with a wavelength of more than 400 nm as well. The transmission rate thereof is not particularly limited.

### (2) Myopia prevention article comprising light emission part

Specific examples of the myopia prevention article that comprises the light emission part that emits ultraviolet light with a wavelength of more than 315 nm and not more than 400 nm, and preferably 360 nm to 400 nm inclusive, according to the present invention include lighting equipment (room lights, lights inside vehicles, lights inside airplanes, street lights, floor lamps, spotlights, and the like), display screens provided to various display devices (for example, televisions, PC monitors, gaming devices, portable media players, cell phones, tablet terminals, wearable devices, 3D glasses, virtual glasses, mobile book readers, car navigation systems, digital cameras, monitors inside vehicles, monitors inside airplanes, and the like), and ultraviolet light irradiation devices used for myopia prevention, but the present invention is not limited thereto.

The light emission part may further produce light with a wavelength of more than 400 nm, but a light emission part that does not produce such light is preferred. Further, the light emission part preferably does not produce ultraviolet light with a wavelength of 315 nm or less (in a case where the light emission part that emits ultraviolet light with a wavelength of more than 315 nm and not more than 400 nm is included) or ultraviolet light with a wavelength of 360 nm or less (in a case where the light emission part that emits ultraviolet light with a wavelength of 360 nm to 400 nm inclusive is included).

The strength of the ultraviolet light with a wavelength of more than 315 nm and not more than 400 nm, and preferably 360 nm to 400 nm inclusive, emitted by the light emission part is not particularly limited, but is preferably 5.0 mW/cm² or less, and more preferably 3.0 mW/cm². The strength of the emitted light can be measured using a known method.

Note that the myopia prevention article that comprises the light emission part according to the present invention may also use with the myopia prevention article that comprises the light transmission part described above, and thus a more superior myopia prevention effect is exhibited more safely.

### (3) Myopia prevention method

A myopia prevention method according to the present invention is mounting the myopia prevention article that comprises the light transmission part described above. The mounting method is not particularly limited, and thus the myopia prevention article may be mounted as appropriate in accordance with the article type.

Further, another myopia prevention method according to the present invention is using the myopia prevention article that comprises the light emission part described above. The method of use is not particularly limited, and thus the myopia prevention article may be used as appropriate in accordance with article type. If the myopia prevention article is a daily necessity such as a room light, the room light may be simply used in daily life without a special method of use. Further, if the article is an ultraviolet light irradiation device used for myopia prevention, the article may be used so that the light irradiated from the irradiation device enters the eye for a certain period of the day.

Further, yet another myopia prevention method of the present invention is mounting the myopia prevention article that comprises the light transmission part described above while using the myopia prevention article that comprises the light emission part described above. According to this method, a more superior myopia prevention effect is exhibited more safely.

Note that these methods can be applied to a human as well as a vertebrate other than a human.

### (4) Method for investigating ultraviolet light suitable for myopia prevention

The method for identifying ultraviolet light suitable for myopia prevention is a method for investigating if ultraviolet light with a predetermined wavelength within the range of more than 315 nm and not more than 400 nm, and preferably 360 nm to 400 nm inclusive, can prevent myopia, and includes the step of irradiating ultraviolet light with that wavelength on a human or a vertebrate other than a human. Then, whether or not such a wavelength can actually prevent myopia is investigated using the method set forth in Example 2, for example. This makes it possible to identify which wavelength or wavelength range within the range of more than 315 nm and not more than 400 nm, and preferably 360 nm to 400 nm inclusive, has a particularly high effect.

### Examples

### <Example 1>

Using a phakic intraocular lens as an example of the myopia prevention article that comprises the light transmission part through which ultraviolet light with a wavelength of more than 315 nm and not more than 400 nm is transmitted and ultraviolet light with a wavelength of 315 nm or less is not transmitted, the myopia prevention effect was verified by the test described below.

First, the axial length of the eye was measured, and then the phakic intraocular lens Artisan (brand name) Model 204 (Ophtec BV) through which ultraviolet light is essentially not transmitted across the full spectrum of wavelengths, or the phakic intraocular lens Artiflex (brand name) Model 401 (Ophtec BV) through which ultraviolet light only made of a wavelength in the range of from about 350 to 400 nm is transmitted was surgically inserted into the measured eye. Subsequently, the elongation of the axial length of the eye was measured five years after the insertion procedure. The axial length of the eye was measured using IOL Master (Carl Zeiss Meditec) by a standard method.

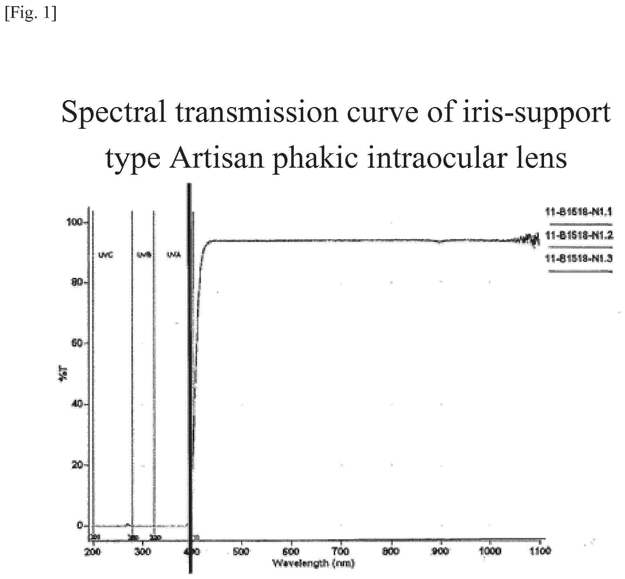

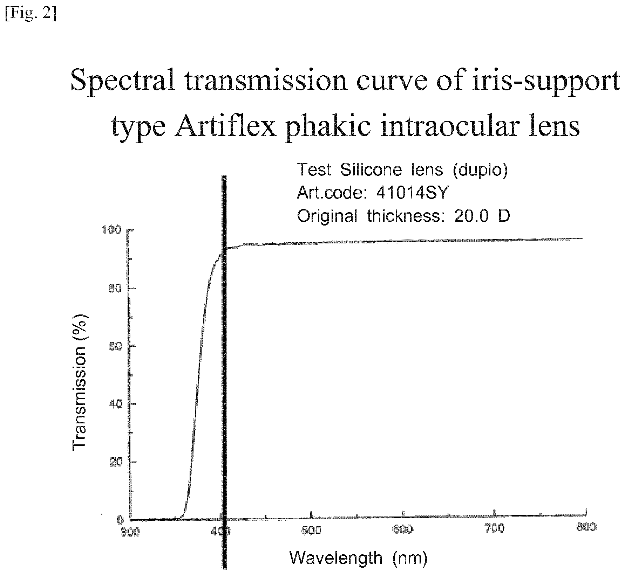

Fig. 1 shows the Artisan (brand name) spectral transmission curve, and Fig. 2 shows the Artiflex (brand name) spectral transmission curve.

Meanwhile, as control eyes, the degrees of elongation over a two-year period of the axial lengths of 185 eyes with high myopia that have not undergone refractive surgery were measured, and the average value was found. The elongation of the axial lengths of the control eyes was 0.065 mm/year on average (for details, refer to Saka N, et al., Graefes Arch Clin Exp Ophthalmol, Vol. 251, pp. 495-499, 2013). The axial length of the eye was measured using the same IOL Master as described above.

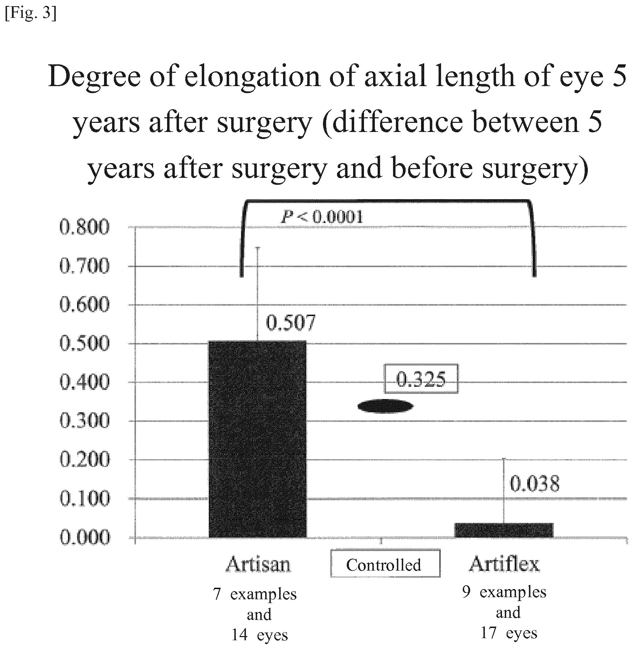

Fig. 3 shows the degrees of elongation over a five-year period of the axial lengths of 7 examples and 14 eyes (average age: 35.7 years) that used Artisan (brand name), 9 examples and 17 eyes (average age: 36.1 years) that used Artiflex (brand name), and the control eyes (185 eyes; average age: 48.4 years). For the eyes that used Artisan (brand name) and the eyes that used Artiflex (brand name), the difference in the axial length of the eye before and after the insertion procedure was set as the degree of elongation of the axial length over the five-year period. For the control eyes, the value obtained by multiplying the average elongation of the axial length over one year found by the above by 5 was set as the degree of elongation of the axial length over the five-year period.

As shown in Fig. 3, the degree of elongation of the axial length of the eye exposed to ultraviolet light only made of a wavelength in the range of from about 350 nm to 400 nm was significantly smaller than that of the eye that was essentially not exposed to ultraviolet light across the full spectrum of wavelengths.

### <Example 2>

Using a UVA irradiation device as an example of the myopia progression prevention article that comprises the light emission part that emits ultraviolet light with a wavelength of more than 315 nm and not more than 400 nm, the myopia prevention effect was verified by the test described below.

A chick is known to develop myopia in an eye (shielded eye) that is covered with a transparent hemisphere (refer to Seko, et al., Invest. Ophthalmol. Vis. Sci, May 1995, Vol. 36, No. 6, 1183-1187, for example). Hence, one eye of each of 30 White-Leghorn chicks was covered with a transparent hemisphere six days after birth. The chicks were divided into a non-UVA irradiation group of 15 that were not subjected to UVA irradiation and a UVA irradiation group of 15 that were subjected to UVA irradiation, and raised for one week spending 12 hours in the light and 12 hours in the dark each day. The degree of myopia development of the shielded eye was then examined.

The UVA irradiation was performed using the UV lamp PL-S TL/08 (Phillips) at a UVA output of 1.7 W.

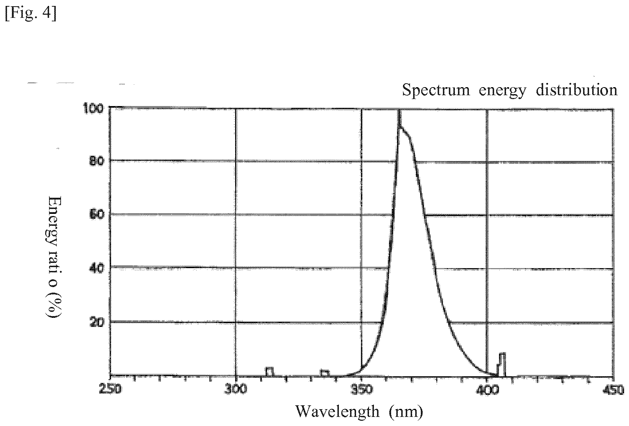

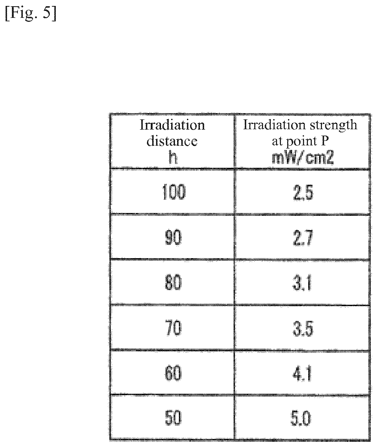

Fig. 4 shows the spectrum energy distribution of the UVA irradiation device, and Fig. 5 shows the relationship between the strength of the irradiated ultraviolet light and the distance from the lamp.

The refractive value, vitreous chamber length, and the axial length of the shielded eye of each of 14 chicks in the non-UVA irradiation group and 13 chicks in the UVA irradiation group were measured 13 days after birth (one week after shielding was started). The refractive value was measured using an autorefractometer by a standard method. The vitreous chamber length and the axial length of the eye were measured using US-4000 (Nidek) in mode B.

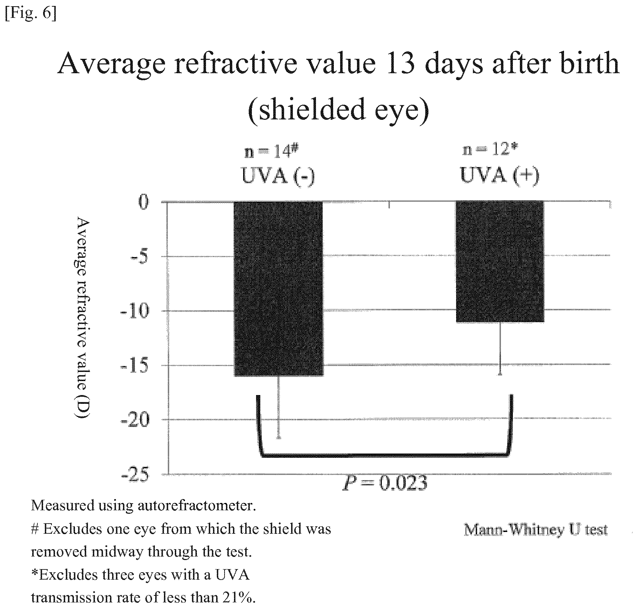

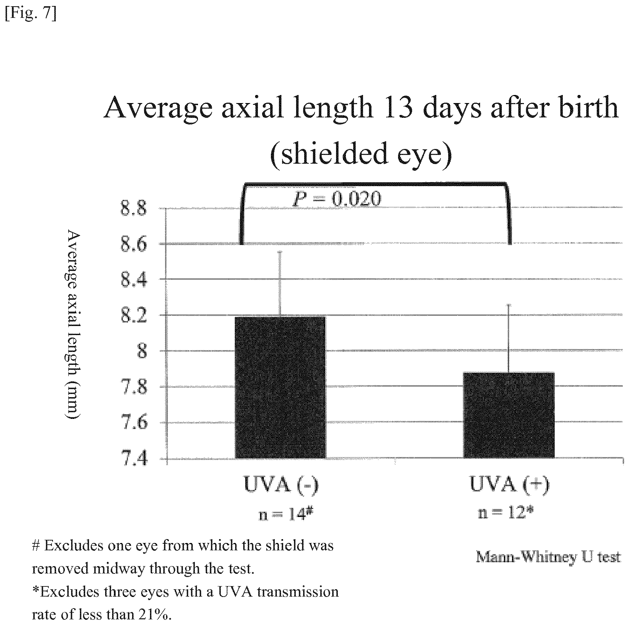

Fig. 6 shows the results of refractive value measurements, and Fig. 7 shows the results of axial length measurements. A Mann-Whitney U test was used for the significance test.

As shown in Fig. 6, the average refractive value of the shielded eyes of the UVA irradiation group was significantly large compared to the average refractive value of the shielded eyes of the non-UVA irradiation group (there was a myopia prevention effect from UVA irradiation). Further, as shown in Fig. 7, the average axial length of the shielded eyes of the UVA irradiation group was significantly small compared to the average axial length of the shielded eyes of the non-UVA irradiation group. Thus, it was clear that the degree of myopia of the UVA irradiation group was significantly small compared to that of the non-UVA irradiation group. Further, based on the values of strength shown in Figs. 4 and 5, the myopia prevention effect was achieved by relatively weak UVA.

### <Example 3>

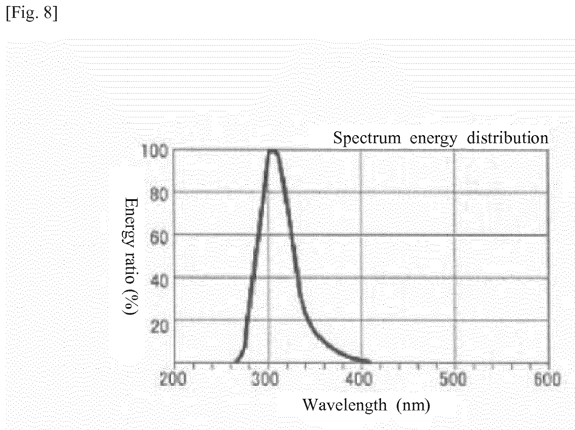

When short wavelength light (Fig. 8) having a peak at 305 nm was irradiated for two days on chicks five days after birth, epithelium inflammation was formed on the corneas of the chicks. Thus, short wavelength light has potent tissue damage properties, and thus the light irradiated for myopia prevention is preferably 340 nm or greater, more preferably 350 nm or greater, and even more preferably 360 nm or greater.

### <Example 4>

To achieve a myopia suppression effect by short wavelength light with a wavelength of more than 315 nm and not less than 400 nm, it is effective to use a lamp that emits light with that wavelength and eyeglass lenses through which that light is transmitted, in combination.

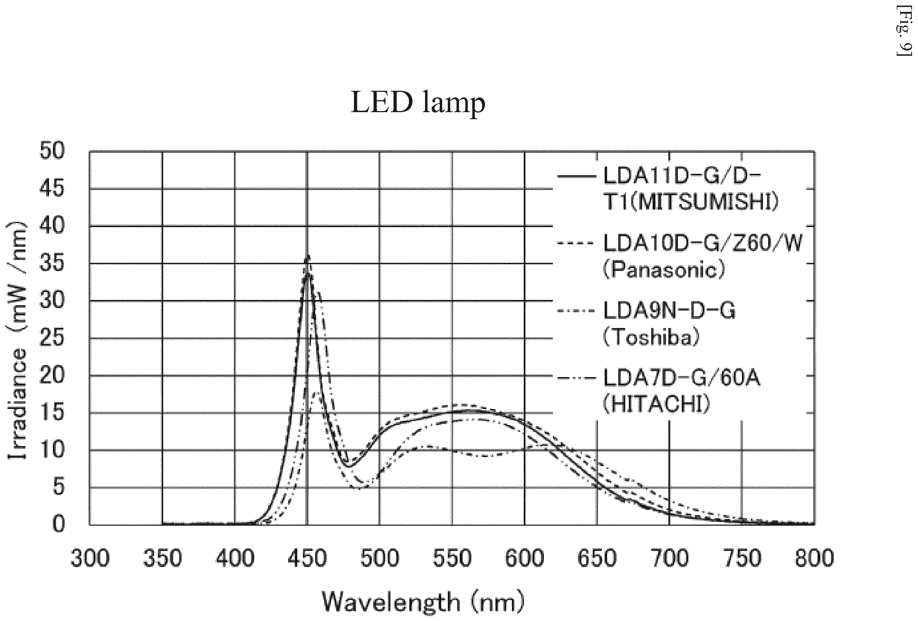

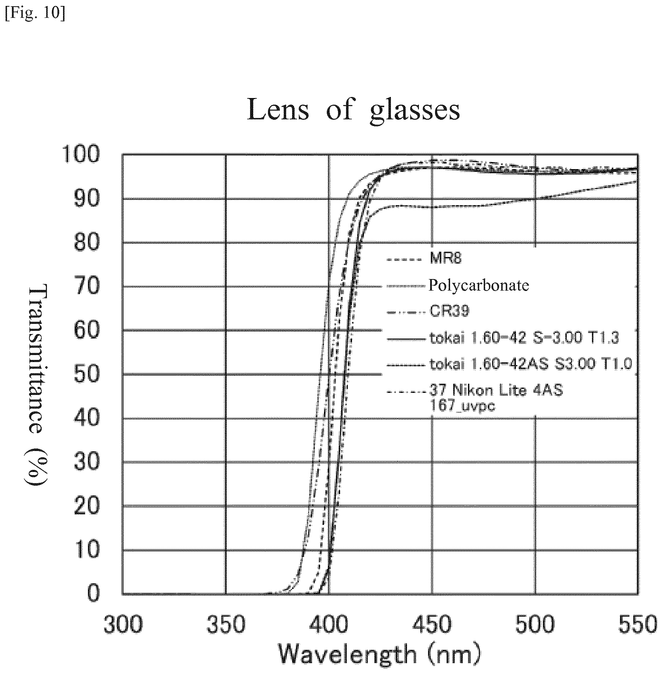

The lamps and glasses already on the market do not exhibit the effect claimed in the present patent. For example, Fig. 9 shows the optical spectrum of an LED lamp that does not emit short wavelength light with a wavelength of more than 315 nm and not more than 400 nm and does not have a myopia suppression effect, and Fig. 10 shows a transmittance light spectrum of eyeglass lenses through which the short wavelength light is not transmitted and by which there is no myopia suppression effect.

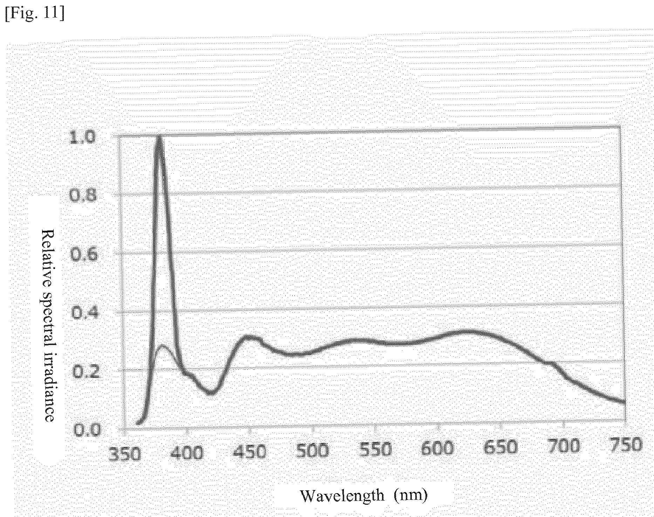

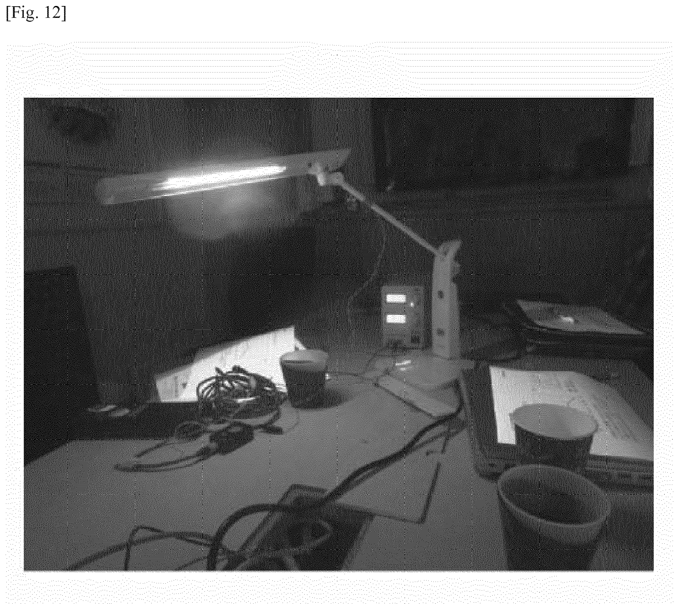

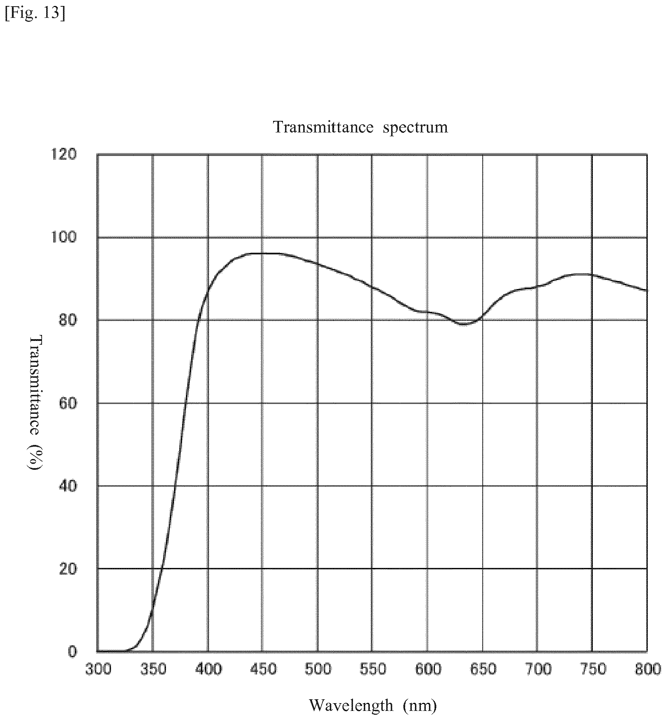

On the other hand, Fig. 11 shows an optical spectrum of a lamp and Fig. 12 shows an image of the lamp as an example of a myopia prevention article of the present invention. The lamp shown in Fig. 12 only uses a special LED that emits the short wavelength light effective for myopia prevention so that the light is easier to see. Further, the lamp in Fig. 12 is capable of changing the strength of the 380 nm serving as the peak wavelength, as shown in Fig. 13.

Fig. 11 shows a transmittance spectrum of the eyeglass lenses through which the short wavelength light effective for myopia prevention is transmitted. Combined use of the glasses in Fig. 13 and the lamp shown in Fig. 11 makes it possible to effectively exhibit the myopia suppression effect.

### <Conclusion>

When the eye is exposed to ultraviolet light with a wavelength of more than 315 nm and not more than 400 nm, and preferably 360 nm to 400 nm inclusive, it is possible to prevent the occurrence of myopia in the eye and slow down the progression of myopia.

### Industrial Applicability

According to the present invention, it becomes possible to provide a myopia prevention article.

## Claims

1. A myopia prevention article (excluding Artiflex (brand name)), comprising a light transmission part through which ultraviolet light with a wavelength of more than 315 nm and not more than 400 nm is transmitted and ultraviolet light with a wavelength of 315 nm or less is not transmitted.

2. A myopia prevention article (excluding Artiflex (brand name)), comprising a light transmission part through which ultraviolet light with a wavelength of 360 nm to 400 nm inclusive is transmitted and ultraviolet light with a wavelength of 360 nm or less is not transmitted.

3. The myopia prevention article according to claim 1 or 2, wherein
the light transmission part further transmits light with a wavelength of more than 400 nm.

4. The myopia prevention article according to any one of claims 1 to 3, wherein
a transmission rate of the ultraviolet light with the wavelength of 315 nm or less is 1% or less.

5. The myopia prevention article according to claim 1, wherein
a strength of the transmitted ultraviolet light with the wavelength of more than 315 nm and not more than 400 nm is 5.0 mW/cm² or less.

6. The myopia prevention article according to claim 2, wherein
a strength of the transmitted ultraviolet light with the wavelength of 360 nm to 400 nm inclusive is 5.0 mW/cm² or less.

7. The myopia prevention article according to any one of claims 1 to 6, wherein
the myopia prevention article is an eyesight correcting tool, an eye protection tool, a face protection tool, a sunshade, a display device, a window, a wall, a light source covering, or a coating material.

8. A myopia prevention article, comprising a light emission part that emits ultraviolet light with a wavelength of more than 315 nm and not more than 400 nm.

9. A myopia prevention article, comprising a light emission part that emits ultraviolet light with a wavelength of 360 nm to 400 nm inclusive.

10. The myopia prevention article according to claim 8 or 9, wherein
the light emission part further emits light with a wavelength of more than 400 nm.

11. The myopia prevention article according to any one of claims 8 to 10, wherein
a strength of the ultraviolet light emitted from the light emission part is 5.0 mW/cm² or less.

12. The myopia prevention article according to any one of claims 8 to 11, wherein
the myopia prevention article is lighting equipment, a display device, or an ultraviolet light irradiation device.

13. A method for investigating if ultraviolet light with a predetermined wavelength within a range of more than 315 nm and not more than 400 nm can prevent myopia, the method comprising a step of irradiating the ultraviolet light with a wavelength of more than 315 nm and not more than 400 nm on an animal.

14. A method for investigating if ultraviolet light with a predetermined wavelength within a range of 360 nm to 400 nm inclusive can prevent myopia, the method comprising a step of irradiating the ultraviolet light with a wavelength of 360 nm to 400 nm inclusive on an animal.

15. A set of the myopia prevention article according to any one of claims 1 to 7 and the myopia prevention article according to any one of claims 8 to 12.

16. The set according to claim 15, comprising a myopia prevention article selected from the group including an eyesight correcting tool, an eye protection tool, a face protection tool, a sunshade, a display device, a window, a wall, a light source covering, and a coating material, and a myopia prevention article selected from the group including lighting equipment, a display device, and an ultraviolet light irradiation device.
